# EUROPÄISCHE PATENTSCHRIFT

(11) **EP 1 666 022 B1**
(45) Veröffentlichungstag und Bekanntmachung des Hinweises auf die Patenterteilung: **14.09.2011**
(21) Anmeldenummer: 05016628.9
(22) Anmeldetag: 30.07.2005
(51) Int. Cl.: A61K 8/86, A61Q 1/04, A61Q 1/08, A61Q 1/10

(54) **Wässrige Zubereitung zur Färbung der Haut**
Aqueous composition for colouring skin
Composition aqueuse pour la coloration de la peau

(30) Priorität: 03.12.2004 DE 202004018813 U
(43) Veröffentlichungstag der Anmeldung: 07.06.2006
(73) Patentinhaber: Faber-Castell AG, 90546 Stein (DE)
(72) Erfinder: Appel, Tatjana, 90522 Oberasbach (DE); Lugert, Gerhard, Dr., 90431 Nürnberg (DE); Appel, Reiner, 90522 Oberasbach (DE)
(74) Vertreter: Mörtel & Höfner

(56) Entgegenhaltungen:
- EP-A- 1 153 597
- WO-A-01/41718
- WO-A-01/41723
- WO-A-03/017956
- SÜD-CHEMIE: "Süd-Chemie Pure Thix and Skin Products" INTERNET ARTICLE, [Online] XP002371339 Gefunden im Internet: URL:http://www.sud-chemie.com/scmcms/web/b inary.jsp?nodeId=5156&binaryId=4309&previe w&disposition=inline&lang=es> [gefunden am 2006-03-09]
- ANONYMOUS: "PURE THIX ? Novel Nonionic Associative Thickeners for Cosmetics and Personal Care" INTERNET ARTICLE, [Online] XP002371340 Gefunden im Internet: URL:http://www.in-cosmetics.com/ExhibitorL ibrary/297/Pure_Thix_associative_thickener .pdf> [gefunden am 2006-03-09]

## Beschreibung

Die Erfindung betrifft eine wässrige Zubereitung zur Färbung der Haut. Die Applikation solcher Flüssigkeiten erfolgt vielfach mit kapillaren Auftragsgeräten. Als Farbmittel müssen lösliche Farbstoffe verwendet werden, da Pigmentfarbstoffe das Kapillarsystem des Auftragsgerät verstopfen würden. Bei Anwendungen in der Kosmetik sollen Farbaufträge einerseits permanent, also lang anhaltend und wischfest sein, sich andererseits aber auch leicht wieder entfernen lassen, etwa nach einem Fehlversuch beim Schminken oder beim Abschminken. Es ist bekannt, dass Farbstoffe, im vorliegenden Fall also wasserlösliche Farbstoffe, an vielen Stoffen beispielsweise unter Ausbildung von Wasserstoffbrücken fest haften bleiben und aufgrund ihrer gelösten Form mit dem Lösungsmittel als Träger leicht in mikroporöse Strukturen eindringen. Deswegen lassen sich solche Farbaufträge von der Haut oft nur mit Mühen und vielfach selbst unter Einsatz von Seife oder spezieller Reinigungslotionen nicht vollständig entfernen, so dass ein Farbauftrag erst nach Tagen völlig verschwunden ist.

Aufgabe der Erfindung ist es, wässrige, lösliche Farbstoffe enthaltende Zubereitungen mit Hilfe von kapillaren Auftragsgeräten durchgeführten Färbung der Haut vorzuschlagen, mit denen sich lang anhaltende und wischfeste, aber leicht wieder entfernbare Farbaufträge erzielen lassen.

Diese Aufgabe wird nach Anspruch 1 dadurch gelöst, dass eine Zubereitung der eingangs genannten Art Polyether-1 mit einem Anteil von 0,02 % bis 1 % enthält und eine Viskosität < 20 mPa s (20°C, Brookfield) aufweist.

Es hat sich überraschenderweise gezeigt, dass der üblicherweise bei Cremes und Pasten, etwa bei aus EP 1 153 597 A2, WO 01/41723 A1, WO 01/41718 A1 und WO 03/017956 A2 bekannten Haarfärbemitteln als Verdicker eingesetzte und bei dünnflüssigen Zubereitungen an sich nicht erforderliche, ja geradezu kontraproduktiv erscheinende Polyether-1 (CAS-Nr. 415696-59-2) die oben beschriebenen nachteiligen Effekte von Farbstoffen verhindert, so dass Farbzubereitungen herstellbar sind, die sich Oberflächen mit mikroporöser Struktur, vor allem von der Haut wieder vollständig, gegebenenfalls unter Verwendung von Seife oder üblichen Reinigungslösungen wieder entfernen lassen. Die dafür ausschlaggebende Substanz Polyether-1 (im folgenden kurz PE-1 genannt) scheint eine große Affinität zu den Farbstoffe aufzuweisen und diese an sich zu binden. Dies dürfte verhindern, dass Farbstoffe kurz nach dem Auftragen, wenn der Farbauftrag also noch feucht ist, in die Oberfläche eindringen. Nach dem Abtrocknen des Farbauftrags bildet sich auf der Oberfläche eine Art Film, in dem die Farbstoffe eingeschlossen sind. Die sich bildenden Filme fühlen sich auf der Haut weich und angenehm an, was unter anderem darauf zurück zuführen sein dürfte, dass keinerlei Spannungen erzeugt werden. Es lassen sich somit vor allem in der Kosmetik anwendbare stark färbende, transparente, permanente und leicht wieder entfernbare gleichmäßige Färbungen erzielen, die mit kapillaren Auftragsgeräten applizierbar sind. Schließlich war noch überraschend, dass Farbaufträge mit einem gegenüber bekannen Zubereitungen für vergleichbare Zwecke erhöhten Glanz erzeugt werden konnten.

Eine Rahmenrezeptur, die sich für eine Vielzahl kosmetischer Anwendungen eignet, setzt sich zusammen aus 0,02 % - 1 % PE-1, 0,05 % - 20 % Farbstoff, 1 % - 30 % Feuchthaltemittel, 0,1 % - 20 % Additive und Wasser ad 100 %.

Unter Additiven sind z.B. Konservierungsmittel, Netzmittel, weitere Verdickungsmittel, weitere Filmbildner, Pflegemittel, Aromen, Vitamine, evtl. auch Lichtschutzfilter zu verstehen. Mit den Netzmitteln lässt sich die Viskosität der Zubereiturigen bei ein und demselben Gehalt an PE-1 variieren.

Als Feuchthaltemitteln können Glycerin, Glykole wie 1-2-Propandiol, 1-2-Butandiol, 1-3-Butandiol, Alkohole oder Zucker enthalten sein. Außerdem können zusätzliche Lösemittel wie Ethanol verwendet werden.

Die Herstellung der erfindungsgemäßen Zubereitungen erfolgt unter Zuhilfenahme von Flügelrührern oder Disolvern und geeigneten Kesseln. In vielen Fällen kann eine moderate Temperaturzuführung, d.h. ein Erwärmen auf 40 - 60°C eine Auflösung der Inhaltsstoffe in der wässrigen Matrix begünstigen. Die Viskosität ist auf Werte < 20 mPa s (20°C Brookfield) eingestellt.

### Beispiel 1:

### Flüssigkeit zum Einfärben der Lippen mit einer Viskosität

| | |
|---|---|
| 1-3-Butandiol (Feuchthaltemittel) | 20,0 Gew% |
| Euxyl K 300¹⁾ (Konservierungsmittel) | 1,0 Gew% |
| Tagat S²⁾ (Emulgator) | 3,0 Gew% |
| Abil B 8843 (Tensid)³⁾ | 1,0 Gew% |
| Ceramide A 2 (Pflegesubstanz)⁴⁾ | 2,0 Gew% |
| CI 45410 | 1,5 Gew% |
| Polyether- 1⁵⁾ | 0,4 Gew% |
| Aroma Vanille (346142)⁶⁾ | 0,5 Gew% |
| Wasser | 70,6 Gew% |
| | 100,0 Gew% |

### Beispiel 2:

### Braun gefärbte Flüssigkeit für Eyeshadow-Anwendungen.

| | |
|---|---|
| Ethanol | 5,0 Gew% |
| 1-2-Propandiol | 15,0 Gew% |
| Euxyl K 300 | 1,0 Gew% |
| Abil B 8851³⁾ | 1,0 Gew% |
| Polyether-1⁵⁾ | 0,5 Gew% |
| C.I. 16035 | 1,2 Gew% |
| C. I. 42090 | 0,08 Gew% |
| C. I. 19140 | 0,15 Gew% |
| D-Panthenol (Pflegesubstanz) | 0,20 Gew% |
| Wasser | 76,87 Gew% |
| | 100,0 Gew% |

Sämtliche Prozentangaben sind Gewichtsprozent.

### Herstellerangaben:

1) Schülke & Mayr GmbH, D-22851 Norderstedt
2) Degussa AG, Essen
3) Degussa AG, Essen
4) Sederma GmbH, D-41334 Nettetal
5) Süd-Chemie AG, München
6) Kurt Georgii, D-71043 Böblingen

## Patentansprüche

1. Wässrige, einen wasserlöslichen Farbstoff enthaltende kosmetische Zubereitung zum Färben der Haut,
**dadurch gekennzeichnet,**
**dass** sie Polyether-1 mit einem Anteil von 0,02 % bis 1 % enthält und eine Viskosität < 20 mPa s (20°C, Brookfield) aufweist.

2. Zubereitung nach Anspruch 1, **gekennzeichnet durch** folgende Zusammensetzung:
| | |
|---|---|
| Polyether-1 | 0,02 - 1 % |
| Farbstoff | 0,05 - 20 % |
| Feuchthaltemittel | 1 - 30 % |
| Additive | 0,1 - 20 % |
| Wasser | ad 100 % |

3. Zubereitung nach Anspruch 1, **gekennzeichnet durch** folgende Zusammensetzung:
| | |
|---|---|
| Polyether-1 | 0,02 - 1 % |
| Farbstoff | 1 - 20 % |
| Additive | 0,1 - 10 % |
| Wasser | ad 100 % |

4. Zubereitung nach einem der Ansprüche 1 bis 6, **dadurch gekennzeichnet, dass** als Additiv ein Tensid enthalten ist.

5. Kapillares Auftragsgerät zum Färben der Haut,
**dadurch gekennzeichnet,**
**dass** es einen mit einer Zubereitung gem. einem der Ansprüche 1 bis 4 gefüllten Flüssigkeitsspeicher enthält.

## Claims

1. Aqueous cosmetic preparation which comprises a water-soluble dye and is for coloring the skin,
**characterized in that**
it comprises polyether-1 in an amount of from 0.02% to 1% and has a viscosity of < 20 mPa s (20°C, Brookfield).

2. Preparation according to Claim 1, **characterized by** the following composition:
| | |
|---|---|
| Polyether-1 | 0.02 - 1% |
| Dye | 0.05 - 20% |
| Humectant | 1 - 30% |
| Additives | 0.1 - 20% |
| Water | ad 100% |

3. Preparation according to Claim 1, **characterized by** the following composition:
| | |
|---|---|
| Polyether-1 | 0.02 - 1% |
| Dye | 1 - 20% |
| Additives | 0.1 - 10% |
| Water | ad 100% |

4. Preparation according to one of Claims 1 to 3, **characterized in that** a surfactant is present as additive.

5. Capillary application device for coloring the skin,
**characterized in that**
it comprises a liquid reservoir filled with a preparation according to one of Claims 1 to 4.

## Revendications

1. Préparation cosmétique aqueuse de coloration de la peau contenant un colorant soluble dans l'eau,
**caractérisée**
**en ce qu'**elle contient un polyéther-1 en une proportion de 0,02 % à 1 % et a une viscosité < 20 mPa s (20°C, Brookfield).

2. Préparation suivant la revendication 1, **caractérisée par** la composition suivante :
| | |
|---|---|
| polyéther-1 | de 0,02 à 1 % |
| colorant | de 0,05 à 20 % |
| agent de conservation de l'humidité | de 1 à 30 % |
| additif | de 0,1 à 20 % |
| eau | pour faire 100 %. |

3. Préparation suivant la revendication 1, **caractérisée par** la composition suivante :
| | |
|---|---|
| polyéther-1 | de 0,02 à 1 % |
| colorant | de 1 à 20 % |
| additif | de 0,1 à 10 % |
| eau | pour faire 100 %. |

4. Préparation suivant l'une des revendications 1 à 3, **caractérisée en ce qu'**elle contient un agent tensioactif comme additif.

5. Appareil d'application capillaire pour la coloration de la peau,
**caractérisé**
**en ce qu'**il contient un réservoir de liquide empli d'une préparation suivant l'une des revendications 1 à 4.
